Europäisches Patentamt

European Patent Office

Office européen des brevets

(19)

(11) Publication number: **0 157 613**
A2

# (12) EUROPEAN PATENT APPLICATION

(21) Application number: 85302219.2

(22) Date of filing: 29.03.85

(51) Int. Cl.⁴: **C 12 P 21/00,** C 12 N 5/00, C 12 N 15/00, G 01 N 33/577, G 01 N 33/574 // (C12P21/00, C12R1:91)

(30) Priority: 30.03.84 US 595075
30.03.84 US 595073

(43) Date of publication of application: 09.10.85
Bulletin 85/41

(84) Designated Contracting States: **AT BE CH DE FR GB IT LI LU NL SE**

(71) Applicant: **SYNTEX (U.S.A.) INC., 3401 Hillview Avenue, Palo Alto, California 94304 (US)**
Applicant: **THE BOARD OF TRUSTEES OF THE LELAND STANFORD JUNIOR UNIVERSITY, Encina 105 Stanford University, Stanford, California 94305 (US)**

(72) Inventor: **Morhenn, Vera Beatrix, 731 DeSoto Avenue, Palo Alto California 94303 (US)**
Inventor: **Schreiber, Alain Benno, 233 C Red Oak Drive East, Sunnyvale California 94086 (US)**
Inventor: **Aillson, Anthony Clifford, 2513 Hastings Drive, Belmont California 94002 (US)**

(74) Representative: **Armitage, Ian Michael et al, MEWBURN ELLIS & CO. 2/3 Cursitor Street, London EC4A 1BQ (GB)**

(54) Monoclonal antibodies specific for human basal cell and malignant squamous cell protein, hybridomas therefor, malignancy test methods and diagnostic kits.

(57) The present invention is concerned with novel monoclonal antibodies specific for an antigenic site on a protein characteristic of a human basal cell and a malignant squamous cell. The antibodies do not bind to mesenchymal cells such as fribroblasts and endothelial cells. The protein on the cell surface which binds to one of the antibodies has a molecular weight of about 120,000 as determined by one dimensional gel electrophoresis. The antibodies find use in diagnostic methods such as the detection of malignant cells, e.g., the detection of residual tumor cells in skin subjected to microscopically-controlled surgery. The invention is also concerned with the use of such antibodies in determining the presence of a malignant condition in exfoliate cell specimens, thus having utility, for example, in the detection of cervical carcinoma.

ACTORUM AG

-1-

## MONOCLONAL ANTIBODIES SPECIFIC FOR HUMAN BASAL CELL AND MALIGNANT SQUAMOUS CELL PROTEIN, HYBRIDOMAS THEREFOR, MALIGNANCY TEST METHODS AND DIAGNOSTIC KITS

Mohs' chemosurgery or microscopically-controlled surgery (MCS) described in Arch. Surg. 42:279-295(1941) and the fresh tissue modification described by Tromovitch, et al. in Arch. Dermatol. 110:231-232 (1974) are used extensively to treat squamous cell carcinomas and certain types of basal cell carcinomas of the skin. These carcinomas are characterized by strands and nests of tumor cells which are not visible on the skin surface. Conventional forms of surgery and radiation often fail to eradicate these silent extensions of tumor so that relatively high recurrence rates result. In MCS, the clinically visible tumor is removed and the next layer of tissue is excised, immediately frozen, cut on a cryostat, stained with hematoxylin and eosin, and checked for residual tumor cells with a light microscope.

MCS is the treatment of choice in squamous cell carcinomas and recurrent basal cell carcinomas. With this form of therapy the cure rate for recurrent basal cell carcinomas is about 90% whereas the cure rate with other forms of therapy (e.g., radiation, conventional surgical excision) ranges from 50% to 85%. For squamous cell carcinomas MCS results in a 5-year cure rate of 94% in a group of patients who have not received previous treatment. However, in recurrent squamous cell carcinomas the 5-year cure rate is only 76.3%. Unsuccessful treatment of basal cell carcinomas causes increased morbidity, repeated treatments with the possibility of mutilation, and economic losses. Recurrences of squamous cell carcinomas may lead to metastasis, which in Mohs' series of squamous cell carcinomas led to death in 97 patients (4.3%).

Residual and undetected tumor cells are thought to be the main reason for recurrence after MCS. When single cells or small islands of tumor cells remain in the tissue, they are sometimes difficult to distinguish on frozen section with conventional staining methods since they may be confused with inflammatory, nerve, or vascular cells. Thus, a staining technique which clearly defines tumor cells should decrease the rate of recurrence and mortality. Furthermore, a definitive staining technique should lower the need for extensive experience in reading frozen sections and for taking extra tissue at the margins if the presence or absence of tumor cells is in doubt on the frozen sections of already removed skin.

The examination of isolated cells or clumps of cells obtained by scraping or washing cut surfaces of tissues or mucous membranes has long proved a useful device in pathological examinations. Early detection of cervical cancer, achieved in part through screening of women with cytologic examination of a cervical Papanicolaou-type (PAP) smear, has contributed significantly to the major improvements in the management of this cancer during the last 30 years. Papanicolaou studied the cytologic examination of cervical smears in the 1940's and extended his initial observations in the early diagnosis of cancer of the human uterine cervix to many fields of cancer diagnosis.

Although the magnitude of benefit is controversial, it appears clear that routine screening of women at risk using the PAP smear is effective in preventing the invasive form of cervical cancer and mortality from it. The PAP smear has changed little since 1943 when Papanicolaou and Trout reported that the cellular changes that accompany malignant tumors of the female genital tract can be used in the early detection of cervical cancer. In the technique, a spatula is used to scrape the cervix, and the resulting exfoliated cells are smeared onto a microscope slide. These cells are stained using several staining steps, and then the exfoliated cells are examined for the presence of abnormal cells by a specially trained cytotechnologist. Examination of a single slide may take up to fifteen minutes. The cellular changes may clearly indicate a cancer or, more likely, may indicate abnormalities that may be due to early stages of a cancer or to inflammation due to some other cause such as an infection. The cellular changes associated with the early stages of cancer are the most difficult to interpret. For this reason it must also be emphasized that there should be careful correlation between the use of the PAP smear and classical biopsy methods.

The field of exfoliative cytology has been extended beyond vaginal, cervical, and endometrial uterine smears to bronchial and prostatic secretions, gastric and colonic washings, impressions of the surface of tumors or the cut surface of biopsy specimens, especially lymph nodes, and serous fluids.

Bronchial secretions such as sputum may be an important specimen for early detection of lung cancer. Lung cancer accounts for more than 25% of all cancer deaths, is the leading cause of cancer deaths in men and is becoming an increasingly serious problem in women. The incidence of lung cancer is increasing very rapidly, with an increase of sixfold over the last forty years. All of the factors that are suspected to contribute to the increased incidence of lung cancer are expected to continue to be a part of modern life so that early detection and effective treatment of lung cancer are essential if there is to be an impact on the mortality due to this disease. Only about 5% of lung cancer patients are cured. The low cure rate is due to a number of factors, a primary factor being that the cancer is often too widespread at the time of its diagnosis to be effectively treated. Most lung cancers are diagnosed on the basis of clinical symptoms such as cough, dyspnea (difficulty in breathing), chest pain, hemoptysis (blood in the sputum), and wheezing. Lung cancers are detected by physical examination, chest x-ray, or exfoliative cytology from a sputum specimen. The use of sputum cytology for early detection of lung cancer is controversial, and the American Cancer Society guidelines for early cancer detection do not recommend sputum cytology. However, other authorities believe that sputum cytology may be a method of early cancer detection. Many lung cancer patients shed cells that demonstrate increasing degrees of cytologic abnormality for long periods of time before the detection of obvious cancer. This disagreement as to the usefulness of sputum cytology is probably due to the lack of well-controlled studies, to variations in the preparation of the specimens for cytologic examination, and to variations in the interpretation of the cytologic changes.

A microscopically-controlled method of cancer excision is described in Arch. Surg. 42:279-295, 1941. A fresh tissue technique in microscopically-controlled excision of skin tumors is disclosed by Tromovitch, et al., in Arch. Dermatol. 110:231-232, 1974. Continuous cultures of fused cells secreting antibody of predefined specificity is described by Köhler, et al., Nature 265:495-497, 1975. A monoclonal antibody against human basal cells which affects the growth of epidermal cells in vitro is disclosed by Oseroff, et al., in Clin. Res. 30:601A, 1982. A biotin-avidin-horseradish peroxidase method of detection of T and B cell antigens with hybridoma antibodies is described by Warnke, et al., in J. Histochem. Cytochem. 28:771-776, 1980. Selective enrichment of human epidermal cell subpopulations using monoclonal antibodies is reported by Morhenn, et al., in J. Inv. Derm., 81:127s-131s, 1983.

The present invention is concerned with novel monoclonal antibodies specific for an antigenic site on a protein characteristic of human basal cells, both normal and malignant. This antigenic site on the protein is also found on malignant squamous cells and, thus, the antibodies of the invention will also bind to malignant squamous cells. Preferably, these monoclonal antibodies are of the IgG type and bind specifically to a protein having a molecular weight of about 120,000 as determined by one dimensional gel electrophoresis. Preferably, these monoclonal antibodies bind to, but do not inhibit the growth of, normal basal cells. The monoclonal antibodies are secreted by a murine hybridoma.

The invention also concerns certain diagnostic methods employing the monoclonal antibodies of the invention. One such method involves the determination of the presence of tumor cells in a specimen suspected of containing such cells. The specimen is contacted with the monoclonal antibody, which is capable of distinguishing human basal cells and such tumor cells from other cell types which may be present in the specimen. The contact is carried out under conditions for binding of the antibody to the tumor cells. After contact, the presence or absence of binding of the antibody to the cells in the specimen is determined. This binding is related to the presence or absence of tumor cells in the specimen. An example of such a method is the detection of residual tumor cells, e.g., malignant squamous cells, in skin subjected to microscopically-controlled surgery to remove a tumor. Generally, the specimen is contacted with a labeled specific binding partner for the monoclonal antibody. This label is capable of producing a detectable signal.

The present invention is also concerned with a method for determining the likelihood of a malignant condition at a locus of interest in a mammalian host. The invention is based in part on the discovery that an exfoliative cell specimen obtained from the locus contains an antigenic site that is usually only present in the cell specimen during a malignant condition. The exfoliative cell specimen is contacted with an antibody specific for this antigenic site. The contact is carried out under conditions for detectable binding of the antibody to such antigenic site. The presence or absence of binding of the antibody to such antigenic site is observed and is related to the probability of a malignant condition at the locus.

The method of the invention finds utility in the detection of a malignant condition in the cervix, vagina, uterus, bronchus, prostate, gastro-intestinal tract including oral pharynx, mouth, etc., and exfoliative material aspirated from manifest tumors or cysts, the cut surface of biopsy specimens, especially lymph nodes, and serous fluids.

The present invention concerns certain diagnostic methods employing antibodies specific for an antigenic site characteristic of a normal human basal cell. One such method involves the determination of the presence of a malignant condition in mammalian tissue, for example, epithelial tissue and tissue composed in part of epithelial cells; e.g., the determination of the presence of malignant squamous cells in a specimen of such tissue suspected of containing such cells. The term "malignant condition" refers to the presence of displastic including carcinoma in situ, neoplastic, malignant, or tumor cells, or the like. The specimen is contacted or combined with a monoclonal antibody capable of distinguishing human basal cells and malignant squamous cells from other cell types which may be present in the specimen. Usually, normal basal cells are not found in the specimen. The contact is carried out under conditions for binding of the antibody to the malignant squamous cells. After contact, the presence of binding of the antibody to the malignant cells in the specimen is observed. That is, the specimen is examined for immune complexes of the antibody and the antigenic site. This immune complex formation is related to the presence of malignant squamous cells in the specimen if normal basal cells are not present in the tissue. Where antibody binding cells are found in the specimen, the location of the binding cells may also be related to the presence of a malignant condition since the finding of basal cells outside, i.e., below, the basal layer is indicative of a malignant condition.

Monoclonal antibodies useful in the method of the invention may be produced according to the standard techniques of Köhler and Milstein, Nature 265:495-497, 1975. For example, epidermal cells from psoriatic plaques are used as the immunogen. The split thickness skin containing epidermal cells from psoriatic plaques is trypsinized and dispersed cells are obtained. These cells are injected into a mouse and, after a sufficient time, the mouse is sacrificed and spleen cells obtained. The spleen cell chromosomes encoding the base sequences for the desired immunoglobulins are immortalized by fusing the spleen cells with myeloma cells or with lymphoma cells, generally in the presence of a non-ionic detergent, for example, polyethylene glycol. The resulting cells, which include the fused hybridomas, are allowed to grow in a selective medium, such as HAT-medium, and the surviving cells are grown in such medium using limiting dilution conditions. The cells are grown in a suitable container, e.g., microtiter wells, and the supernatant is screened for monoclonal antibodies having the desired specificity.

Various techniques exist for enhancing yields of monoclonal antibodies, such as injection of the hybridoma cells into the peritoneal cavity of a mammalian host, which accepts the cells, and harvesting the ascites fluid. Where an insufficient amount of the monoclonal antibody collects in the ascites fluid, the antibody is harvested from the blood of the host. Various conventional ways exist for isolation and purification of the monoclonal antibodies, so as to free the monoclonal antibodies from other proteins and other contaminants (see Köhler and Milstein, supra).

One such monoclonal antibody useful in the method of the present invention is exemplified by a novel antibody designated VM-2. This monoclonal antibody is specific

0157613

-9-

for an antigenic site on a protein characteristic of human basal cells, both normal and malignant. The antigenic site is also found on malignant squamous cells. The protein has little, if any, associated lipid. When lysates of biosynthetically labeled target cells are immunoprecipitated with VM-2 antibody and the precipates are submitted to sodium dodecyl sulfate-polyacrylamide one-dimensional gel electrophoresis (SDS-PAGE), the protein appears as a doublet (two bands) of molecular weight of about 120,000 daltons. The antibody is of the $IgG_1$ isotype. The VM-2 antibody does not bind to mesenchymal cells, such as fibroblasts or endothelial cells, nor to Staphylococcal Protein A at pH 7.3. The VM-2 antibody is produced by the VM-2 murine hybridoma.

Also included within the scope of the invention are useful binding fragments of the VM-2 monoclonal antibody such as Fab, $F(ab')_2$, Fv, and so forth. The antibody fragments are obtained by conventional techniques. For example, useful binding fragments may be prepared by peptidase digestion of the antibody using papain or pepsin.

While the above specific example of the novel antibodies of the invention is directed to an antibody of the IgG class from a murine source, this is not meant to be a limitation. The above antibody and those antibodies having functional equivalency with the above antibody, whether from a murine source, mammalian source including human, or other sources, or combinations thereof are included within the scope of this invention, as well as other classes such as IgM, IgA, IgE, and the like, including isotypes within such classes. By the term "functional equivalency" is meant that the antibody is capable of binding to the above-described antigenic site and capable of competing with the VM-2 antibody for such site. That is, such antibody, when combined with a specimen containing a cell or cell fragment having such antigenic site, will bind to such antigenic site and will block VM-2 antibody from binding to such site.

Another monoclonal antibody which may be used in the method of the present invention is disclosed in Clin. Res., supra. The disclosed antibody was prepared using epidermal cells obtained from a psoriatic plaque. An antibody secreting hybridoma cell line was produced according to standard techniques. The monoclonal antibody which was produced was termed VM-1. The antibody is an $IgG_1$.

One difference between the VM-1 and the VM-2 antibodies is that the former inhibits the growth of basal cells whereas the latter does not inhibit such growth. Another difference is that the VM-1 monoclonal antibody does not bind to the same human basal cell surface antigen as the VM-2 monoclonal antibody. The antigen to which VM-1 binds is extractable with methanol and chloroform and is probably lipid in nature.

A particular example, by way of illustration and not limitation, of a method in accordance with the invention is a method for the detection of residual tumor cells in microscopically-controlled surgery of the skin. The dermis or deeper layers of the skin, where normal basal cells are usually not found, are examined for tumor cells. The above method is applied to a specimen which is a section of skin obtained after removal of the tumor. The section of skin obtained is adjacent to the tumor and generally is the next layer of tissue which is excised after excision of the tumor or after excision of a previous layer of skin. The layer of skin which is excised is treated to obtain sections of such skin, which treatment initially involves freezing the layer of skin or tissue, normally freezing immediately after excision.

The frozen layer of tissue is then cut into sections using, for example, a cryostat.

The section of skin obtained as described above is contacted with a first monoclonal antibody capable of distinguishing human basal cells and malignant squamous cells from other cell types which may be present in the specimen and then with a second antibody against the above monoclonal antibody, which second antibody is labeled with a detectable label.

The excised specimen, e.g., the section of skin, is contacted with the first monoclonal antibody under conditions for binding of the antibody to malignant tumor cells. The incubation is generally conducted in an aqueous medium such as, for example, phosphate buffered saline containing a small amount of sodium azide, in a suitable container such as, for example, a glass petri dish, for a period from about 15 to 30 minutes at a temperature of from about 20 to 30°C. The amount of antibody employed is usually sufficient to provide detectable binding, i.e., to provide a detectable number of complexes between the antibody and the antigenic site in question.

Following the incubation, the section is washed to reduce or eliminate non-specifically bound antibody and then is examined to observe the above-mentioned complexes which result from binding of the monoclonal antibody to the cells of the specimen possessing the antigenic site. The binding is related to the presence of residual tumor cells in the skin. Accordingly, binding is determined, for example, by contacting the specimen with a labeled specific binding partner for the monoclonal antibody. The label is capable of producing a detectable signal and may be a radioactive label, a chromophore such as a fluorescer, an enzyme, or the like.

An example of a technique employing the above approach is immunofluorescence staining. In this technique frozen sections of skin are fixed on a glass slide with acetone and are incubated with the monoclonal antibody in, for example, a petri dish. After washing with an appropriate buffer such as, for example, phosphate-buffered saline, the skin section is placed on a petri dish and contacted with the labeled specific binding partner for the monoclonal antibody, which may be, for example, a labeled antibody specific for the monoclonal antibody employed. Since, for the most part, the monoclonal antibody will be derived from a murine source, a labeled antimouse immunoglobulin specific for the monoclonal antibody may be employed. Such immunoglobulins may be raised according to standard techniques by injecting a suitable host with murine antibody, waiting for an appropriate time, and harvesting the antimouse immunoglobulins from the blood of the injected host.

After a second washing of the slide with, for example, an aqueous buffer, the sections may be covered with a fluorescent antibody mounting fluid and a coverslip and then examined with a fluorescence microscope to determine the binding of the monoclonal antibody to the skin section. The determination of the binding also may include an identification of the location of such binding within the specimen.

The binding of the monoclonal antibody to the specimen may also be determined by employing a monoclonal antibody which is covalently conjugated to a label capable of producing a detectable signal, such as a radioactive entity, a chromophore including dyes and fluorescers, or an enzyme. The number of labels employed per antibody is generally determined by the requirements of the diagnostic method in which the labeled antibody is employed and the availability of sites for linking the label to the antibody.

Methods for conjugating labels to antibodies and antibody fragments are well-known in the art. Such methods may be found in U.S. Patent Nos. 4,220,450; 4,235,869; 3,935,074; 3,996,345; and 3,817,837.

Another example of a technique in which the monoclonal antibody of the invention may be employed is immunoperoxidase labeling (Warnke, et al., J. Histochem. Cytochem., 28:771-776, 1980). The tissue to be tested is fixed with a suitable solvent, such as acetone, on a support, such as a glass slide. Next, the tissue is incubated with the monoclonal antibody and then washed free of unbound antibody. Then, the tissue is incubated with biotin-conjugated anti-mouse IgG, washed to remove unbound antibody, combined with avidin conjugated horseradish peroxidase, washed to remove unbound conjugate, and then treated with substrate for the enzyme. Following this treatment the slide is examined for a detectable signal.

The present invention also provides for an improvement over the classical method of exfoliative cytology. In this method of the present invention, the presence of a malignant condition in an exfoliative cell specimen obtained from a locus of interest in a mammalian host is determined. This method will next be described in more detail using, for the purpose of illustration and not limitation, a monoclonal antibody that is specific for an antigenic site on a protein characteristic of human basal cells, both normal and malignant. Illustrative of such an antibody is the monoclonal antibody obtained form a murine hybridoma, which is designated VM-2.

It has been found that the VM-2 antibody may suitably be used in this method. Also useful in the method of the invention are fragments of the VM-2 monoclonal antibody such as Fab, F(ab')$_2$, Fv, and so forth. By the term "exfoliative" is meant that the specimen comprises isolated cells or clumps of cells obtained by scraping or washing the surface of tissue, which cells are removed individually or in scales or laminae. The exfoliative cell specimen is to be distinguished from excised tissue such as that obtained by biopsy. The exfoliative cell specimen obtained from the locus is characterized in that a certain antigenic site is usually found in the specimen only when a malignant condition is present. In one embodiment this antigenic site shares determinants with an antigenic site of a normal cell that is not usually expected to be present in the exfoliative cell specimen. The specimen is contacted with an antibody that is specific for the aforementioned antigenic site. The antibody is capable of distinguishing over other cell types which are usually found in the specimen. Contact between the specimen and the antibody is made under conditions for binding of the antibody to the antigenic site. After contact, the presence or absence of binding of the antibody to the antigenic site is determined and is related to the presence of a malignant condition at the locus.

While the VM-2 antibody is referred to above, this is by way of illustration and not limitation. Any antibody may be used if it binds to an antigenic site that is usually found in an exfoliative cell specimen only when a malignant condition exists and is capable of distinguishing over other cell types that are usually found in the specimen. Preferably, the antigenic site shares determinants with an antigenic site on a normal cell that is not usually expected to be present in the exfoliative cell specimen. The antibody may be from a murine source, a mammalian source including human, or other source, or a combination thereof. The antibody is preferably an IgG but may be an IgM, IgE, IgA, or the like.

In this method of the invention, exfoliative cell specimen is obtained from a locus of interest, i.e., a locus on or in a mammalian host, which locus may or may not have a malignant condition. The specimen may be obtained, for example, by scraping or washing of tissue at the locus. The locus may have membranes covered with squamous cells or with non-squamous cells. Depending on the nature of the tissue involved, or the location of the tissue as the case may be, one may collect an exfoliative body fluid, such as, for example, sputum, which body fluid has been in contact with, and may be said to have washed, the tissue at the locus. The exfoliative cell specimen may be obtained in accordance with the usual techniques of exfoliative cytology. In the detection of cervical carcinoma, for example, a scraping from the cervix would be taken. To determine the presence of malignancy in the lung, a sputum sample would provide the exfoliative cell specimen to be used in the present method. The method finds utility in the detection of a malignant condition in exfoliative cell specimens from the cervix, vagina, uterus, bronchus, prostate, gastro-intestinal tract including oral pharynx, mouth, etc., and exfoliative cell specimens taken from impressions of the surface of tumors or cysts, the cut surface of biopsy specimens, especially lymph nodes, and serous fluids.

The exfoliative cell specimen is next contacted with the aforementioned VM-2 antibody under conditions for binding of the antibody to the specific antigenic site in the specimen to form antigen-antibody complexes. This antigenic site may be present on cells or cell fragments in the specimen. Generally, the specimen is placed on an appropriate support, such as, for example, a slide, usually glass, or some other suitable material. The exfoliative cell specimen is generally smeared on the slide to provide a thin layer of the specimen on the surface of the slide. The contact between the antibody and the specimen is generally carried out in an aqueous buffered medium. The buffers which may be employed include phosphate, tris, bicarbonate, etc. The pH is related to the nature of the specimen and the antibody, and is generally in the range of from about 5 to 8. The aqueous medium may additionally contain organic polar solvents in an amount of from about 0 to 40%. The organic polar solvents are water soluble and generally have from about 1 to 10 carbon atoms and from about 1 to 4 oxygen atoms. The antibody will be present in the aqueous medium at a concentration of about 1 to 100 µg/ml, preferably from about 10 to 20 µg/ml. The temperature during the contact of the specimen with the antibody is usually from about 4 to 40°C, preferably about 10 to 30°C. The period of contact is usually from about 15 to 120 minutes, preferably from about 30 to 60 minutes.

After the period of contact between the specimen and the antibody, the support is generally treated to remove unreacted antibody. Normally, this is accomplished by washing the support with an aqueous, usually buffered, medium. In general, the amount of wash solution should be sufficient to remove the unreacted antibody.

Next, the presence of binding of the antibody to the antigenic site in the specimen, which binding is related to the presence of a malignant condition at the locus, is observed. That is, the specimen is examined to determine the number of antigen-antibody (immune) complexes formed. It should be noted that in some instances very small numbers of the antigenic site in question may be found in the exfoliative cell specimen. However, in a malignant condition, large numbers of the antigenic site will be present and this latter condition is readily distinguishable by this method over a non-malignant condition because a large number of antigen-antibody complexes will be measurable where a malignant condition exists. To make the determination of the presence of binding, means for producing a detectable signal is incorporated into the assay system. For example, one may conjugate the antibody employed in the assay to a label which is capable of producing a detectable signal. The label may be a radioactive entity, a chromophore including dyes and fluorescers, an enzyme, or the like. The number of labels employed for the antibody is generally determined by the requirements of the method of the present invention and the availability of sites for linking the label to the antibody.

Alternatively, one may contact the washed slide with a labeled specific binding partner for the antibody, which may be, for example, a labeled antibody specific for the antibody employed. Where the monoclonal antibody is derived from a murine source, a labeled anti-mouse immunoglobulin specific for the antibody employed in the method may be used. Such immunoglobulins may be raised according to standard techniques by injecting a suitable host with the monoclonal antibody, waiting for an appropriate time, and harvesting the anti-mouse immunoglobulins from the blood of the injected host. When a labeled specific binding partner for the antibody is employed, the slide must be washed again with an aqueous medium prior to examining the slide for fluorescence.

To determine the presence of binding between the antibody and the cell specimen where a fluorescer label is used, one may examine the slide for fluorescence, usually employing a fluorescence microscope. Where a label other than a fluorescer is employed, one may examine the slide or the specimen for the formation of a precipitate, a color, or the like.

The above description is directed primarily to the use of the antibodies of the invention in immunofluorescence techniques. However, the antibodies of the invention may be used in most assays involving antigen-antibody reactions. The assays may be homogeneous or heterogeneous. In a homogeneous assay approach, the specimen is lysed and clarified to remove debris. The immunological reaction usually involves the specific antibody, a labeled analyte, and the sample of interest. The signal arising from the label is modified, directly or indirectly, upon the binding of the antibody to the labeled analyte. Both the immunological reaction and detection of the extent thereof are carried out in a homogeneous solution. Immunochemical labels which may be employed include free radicals, fluorescent dyes, enzymes, bacteriophages, coenzymes, and so forth.

In a heterogeneous assay approach, the reagents are usually the specimen, the specific antibody, and means for producing a detectable signal. The specimen is generally placed on a support, such as a plate or a slide, and contacted with the antibody in a liquid phase. The support is then separated from the liquid phase and either the support phase or the liquid phase is examined for a detectable signal employing means for producing such signal. The signal is related to the presence of the analyte in the specimen. Means for producing a detectable signal includes the use of radioactive labels, fluorescers, enzymes, and so forth. Exemplary of heterogeneous immunoassays are the radioimmunoassay, immunofluorescence methods, enzyme-linked immunoassays, and the like.

For a more detailed discussion of the above immunoassay techniques, see "Enzyme-Immunoassay," by Edward T. Maggio, CRC Press, Inc., Boca Raton, Florida, 1980. See also, for example, U.S. Patent Nos. 3,690,834; 3,791,932; 3,817,837; 3,850,578; 3,853,987; 3,867,517; 3,901,654; 3,935,074; 3,984,533; 3,996,345; and 4,098,876, which listing is not intended to be exhaustive.

The invention also includes a diagnostic kit for carrying out the method disclosed above. In one embodiment, the diagnostic kit comprises (a) a monoclonal antibody more specifically defined above and (b) a conjugate of a specific binding partner for the above monoclonal antibody and a label capable of producing a detectable signal. The reagents may also include ancillary agents such as buffering agents and protein stabilizing agents, e.g., polysaccharides and the like. The diagnostic kit may further include, where necessary, other members of the signal producing system of which system the label is a member, agents for reducing background interference in a test, control reagents, apparatus for conducting a test, and the like. In another embodiment, the diagnostic kit comprises a conjugate of a monoclonal antibody of the invention and a label capable of producing a detectable signal. Ancillary agents as mentioned above may also be present.

The antibodies of the invention may be used therapeutically where binding to normal basal cells would not be a problem. For therapeutic use the antibodies are bound to a toxin to form an immunotoxin or to a radioactive material to form a radiopharmaceutical. Methods for producing immunotoxins and radiopharmaceuticals of antibodies are well-known.

The invention also includes a diagnostic kit for carrying out the exfoliate method disclosed above. The diagnostic kit comprises (1) means for obtaining an exfoliative cell specimen, e.g., a swab, and (2) a conjugate of a monoclonal antibody and a label, which conjugate is more specifically defined above. The conjugate reagent may also include ancillary agents such as buffering agents and protein stabilizing agents, e.g., polysaccharides and the like. The diagnostic kit may further include, where necessary, other members of the signal producing system of which system the label is a member, agents for reducing background interference in a test, control reagents, apparatus for conducting a test, e.g., a slide, and the like. In another embodiment, the diagnostic kit comprises (1) a monoclonal antibody more specifically defined above and (2) a conjugate of a specific binding partner of the above monoclonal antibody and a label capable of producing a detectible signal and (3) means for obtaining an exfoliative cell specimen. The kit may further include ancillary agents and, where necessary, other members of the signal producing system. The diagnostic kit may be employed alone or it may be used in conjunction with other tests, such as the standard PAP smear or with tests for other infections, such as, for example, chlamydia, herpes virus, gonorrhea, cytomegalovirus, and so forth.

## EXAMPLES

The invention is further demonstrated by the following illustrative Examples. A number of procedures employed will be described first.

Cellular Enzyme Linked Immunosorbent Assay (ELISA)

(a) Cell lines: Human foreskin fibroblasts (HFF) were established from primary cultures derived from circumcisions; cells were used between transfers 4 to 10. Peripheral blood lymphocytes, mononuclear cells and erythrocytes were obtained from healthy volunteers. The A-431 vulvar carcinoma cell line and A-549 bronchial carcinoma cell line and murine BALB/c 3T3 fibroblasts were obtained from Oncogen, Seattle, Washington. Hela cervical carcinoma cells, GH3 rat pituitary tumor cells, normal rat kidney fibroblasts (NKR), Daudi human B lymphoma, Molt T lymphoma and P 388 Dl murine macrophage cell line were obtained from the ATCC. Skin squamous cell carcinoma (SCC) cell line (termed SCL-1) was obtained from Dr. N. Fusenig, Heidelberg, Germany, and bovine and rabbit aortic endothelial cells (EC) were prepared according to standard techniques. Bovine venous EC were obtained from the University of California at San Francisco, and murine capillary EC from Dr. A. Curtis of Glasgow, Scotland. All tissue culture cells were grown in Dulbecco's minimal essential medium (DMEM, MA Bioproducts) containing 10% fetal calf serum (Hyclone).

(b) Adherent cells were grown to subconfluence in 96 well Linbro dishes: cells growing in suspension were allowed to adhere to the 96 well dishes for 30 min at 37°C after precoating of the wells with 50 μl/well of a 0.1% poly L-lysine (Miles Laboratories, Inc.) solution in phosphate buffered saline (PBS). Cells were then fixed in the wells for 5 min at room temperature with 0.25% glutaraldehyde (Sigma Chemical Corporation) and washed 3 times with PBS. Dishes were either used immediately or stored at 4°C in humidified chambers. Cells were incubated at 37°C for 2 h with monoclonal antibody, washed with PBS containing 0.1% bovine serum albumin (PBS-BSA) and further incubated with rabbit anti-mouse (Ig) immunoglobin antibodies coupled to peroxidase (Zymed or Cappel) at 37°C for 2 h. After washing with PBS-BSA cells were incubated for 10 min at room temperature with 1 μg/ml orthophenylenediamine and 0.03% $H_2O_2$ in 0.1 M citrate buffer, pH 4.5. Optical density (O.D.) at 630 nm of individual wells was determined on a Dynatec ELISA plate reader. O.D. readings tenfold higher than that of controls (no first and/or no second antibody incubation) was considered to reflect significant binding of the antibody to the cells.

Immunofluorescence (IF) Staining of Frozen Section of Skin or Dispersed Cells

The binding of antibodies to epidermal cells in situ was determined by IF using rabbit anti-mouse fluorescein isothiocyanate conjugated Ig (R/M-FITC). This reagent had been absorbed on dispersed human skin cells. Frozen sections of skin were incubated with monoclonal antibody (either VM-1 or VM-2) in humidified glass petri dishes for 15 minutes at room temperature, washed with PBS, labeled with R/M-FITC for 15 minutes and washed with PBS (Harrist & Mihm, Hum. Pathol., 10:625-653, 1979). Sections were covered with fluorescent antibody mounting fluid (DIFCO) and a glass coverslip and examined with a Zeiss fluorescence microscope. Trypsinized cell suspensions were labeled in a similar manner. Aliquots of labeled cells were resuspended in PBS, placed on a slide, covered with a glass cover slip and examined with a fluorescence microscope.

Immunoperoxidase (IP) Labeling of Frozen Skin Sections

Frozen sections of human skin were labeled using the immunoperoxidase staining technique (Warnke et al., J. Histochem. Cytochem., 28:771-776, 1980). Briefly, the tissue was fixed with acetone, incubated with monoclonal antibody (either VM-1 or VM-2), washed with PBS, incubated with biotin-conjugated goat anti-mouse IgG (G/M-IgG)(Tago, Inc.), washed with PBS, labeled with avidin conjugated horseradish peroxidase, washed with PBS and $H_2O$, incubated with fresh diaminobenzidine solution and rinsed with PBS and $H_2O$. All incubations were at room temperature. The sections were processed in 0.5% $CuSO_4$ solution, counterstained with Giemsa, cleared and mounted. Slides were examined with a light microscope.

$^{35}$S-Methionine Labeling of VM-2 Antibody

Hybridoma cells producing VM-2 antibody were seeded into a microtiter well in methionine free DMEM containing 25 mM Herpes buffer, 4 mM L-glutamine, 4.5 gm/l glucose, 10 mM non-essential amino acids, 100 units/ml penicillin, 100 μg/ml streptomycin and 15% heat inactivated newborn calf serum (NCS). The cells were contacted for 6 hrs with 0.1 mCi $^{35}$S methionine at 37°C, the supernatant removed and centrifuged to remove cells.

Isotype Determination of VM-2

(a)     Ouchterlony immunodiffusion

An aliquot of supernatant of VM-2 hydridoma cells was placed into the center well of a 2% agar plate. Monospecific rabbit anti-mouse Ig isotypes antibodies (Meloy) were placed in the outer wells and the plate was incubated for 2 h at room temperature and overnight at 4°C.

(b)     Flexible polyvinylchloride 96 well plates (Costar) were coated with 0.1 mg/ml goat anti-mouse Ig antibodies for 2 h at 37°C and countercoated with a 3% BSA solution for 2 h at 37°C. VM-2 hydridoma supernatant was then incubated at 37°C for 2 h. After washing with PBS-BSA plates were incubated at 37°C for 2 h with monospecific rabbit anti-mouse Ig isotype antibodies coupled to peroxidase (Zymed). After washing, plates were incubated with 1 mg/ml orthophenylenediamine and 0.03% $H_2O_2$ in 0.1 M citrate buffer pH 4.5. Optical density at 630 nm was determined on a Dynatec ELISA plate reader.

Staphylococcal Protein A Binding Assay

Microtiter wells were incubated with 5% NCS in PBS plus 0.02% $NaN_3$ and the supernatant was aspirated. Twenty-five μl of a suspension of epidermal cells ($2\times10^7$ cells/ml) were added to each well and incubated with 25 μl of VM-2 for 1 hr at room temperature. The plates were centrifuged at 1200 rpm for 7 min, washed twice with 50% NCS/PBS/NaN$_3$ and 25 μl $^{125}$I-staphylococcal protein A (about 50,000 cpm/25 1) were added. The plates were incubated for 1 hr at 25°C, washed twice with 5% NCS/PBS/NaN$_3$ and dried. The bottom of the wells were cut off and counted in a gamma counter.

Immunoprecipitation Studies

SCC, A-431, Hela and HFF cells were grown to subconfluence in 100 mm tissue culture dishes in DMEM containing 10% FCS. Cells were incubated at 37°C for 4 h with 100 μCi [$^{35}$S]-methionine in DMEM deficient in methionine (GIBCO) containing 10% dialyzed FCS. Cells were washed with PBS-BSA and lysed with PBS containing 0.5% Triton X-100 (Sigma) for 30 min at 4°C. Lysates were centrifuged for 4 min at 10,000xg in an Eppendorf centrifuge to remove cell nuclei and debris. Lysates were then incubated at 4°C for 2 h with 20 μg VM-2 antibody and antigen-antibody complexes were precipitated with 100 μg goat anti-mouse Ig antibody by overnight incubation at 4°C and centrifugation. Immunoprecipitates were washed 4 times with PBS-BSA containing 0.1% Triton X-100 and solubilized in 20 ml Laemli sample buffer by boiling for 2 min. Antigen analysis was performed on 5-15% acrylamide gradient one dimensional sodium dodecyl sulphate polyacrylamide slab gels. Gels were run at 30 mA constant intensity for 6 h, stained with Coomassie Brilliant Blue, destained, treated with Enhance® (NEN), dried and processed for fluorography for one or two days. Borohydride tritiated protein mixtures were prepared using standard techniques and were run in parallel to allow apparent molecular weight determinations.

## EXAMPLE 1
### Preparation of VM-2 Antibody

A.  Isolation and Culture of Human Epidermal Cells

Single cell suspensions of skin cells were prepared from split thickness skin from psoriatic plaques removed with a keratotome (Davol) preset at 0.015 inches or from skin obtained at surgery (for keratinocyte cultures). Full thickness skin obtained at surgery was trimmed, cut into 1 x 5 mm strips and split-cut with a Castroviejo keratotome set at 0.1 mm. Strips of split-thickness skin were treated for 25 min at 37°C with 0.3% trypsin (ICN Pharmaceuticals) in 0.8% NaCl, 0.04% KCl, 0.1% glucose, pH 7.3, plus 0.1% EDTA. The skin slices were washed, transferred to complete growth medium consisting of Dulbecco's Minimum Essential Medium (DMEM) plus 10% heat inactivated fetal calf serum (FCS), 50 µg/ml gentamicin, 2 mM L-glutamine, 50 units/ml penicillin, 50 µg/ml streptomycin and the basal and malpighian cells were released into the medium by gentle agitation. For culture, $2 \times 10^6$ viable, round refractile cells from normal skin were plated on a collagen thin gel coated 3.5 cm petri dish (Flow Labs) and incubated in 5% $CO_2$:95% air at 37°C. Viability was determined by trypan blue exclusion.

B.  Production of Antibody

Using standard techniques (Köhler and Milstein, supra), MOPC-21 myeloma cells were fused with spleen cells obtained from a BALB/c(NIH strain)mouse. To immunize and boost the mouse, keratotome sections from psoriatic plaques from 2 unrelated donors were incubated in trypsin/EDTA as described above. The dispersed cells were washed once with complete growth medium, resuspended in PBS and injected into the mouse intraperitoneally. The antibodies produced by fused cells were screened by the immunofluorescence technique described above using frozen sections prepared from both normal skin and skin
obtained from psoriatic plaques. Skin for frozen
sections from psoriatic plaques was obtained using a
local anesthetic and a 3 mm biopsy punch.

## EXAMPLE 2

### A.  Characterization of VM-2 Antibody

VM-2 was cloned and then subcloned 2 times. The
last two cultures were derived from microtiter wells for
which serial dilutions predicted 1/2 cells/well. Cells
were grown in large scale in 75 $cm^2$ tissue culture
flasks for 12 h in the absence of fetal calf serum.
Conditioned medium was precipitated with 35% saturated
ammonium sulfate for 4 h at 4°C. Precipitates were
extensively dialyzed against PBS and yielded
10-20 μg/ml medium semi-purified VM-2 antibody.
Alternatively, $10^7$ VM-2 cells were injected
intraperitoneally in pristane treated BALB/c mice. After
10 days ascites fluid was collected, cleared by
centrifugation and precipitated with 35% saturated
ammonium sulfate. After dialysis VM-2 antibody was
further purified by gel chromatography on an LKB Ultragel
AcA-34 column. Ascites fluid yielded between 2-5 mg
antibody/ml. On one dimensional gel electrophoresis, the
antibody consists of two heavy chains of molecular weight
of about 50,000 daltons, and two light chains of
molecular weight about 25,000 daltons. The antibody does
not bind to staphylococcal protein A. On Ouchterlony
immunodiffusion, a precipitin band was seen only in the
area of the antibody against $\gamma_1$ and anti-7s
antibody. Thus, VM-2 is an IgG of the $\gamma_1$ subtype.
The $IgG_1$ nature of VM-2 was also confirmed by the solid
phase double antibody ELISA procedure described above.

VM-2 does not inhibit cellular growth. Cultures
treated with a 1:10 dilution of VM-2 showed about a 4-fold increase in DNA content during the period of culture as did the controls. By contrast in parallel epidermal cell cultures incubated with VM-1 the total DNA content/plate stayed constant or decreased slightly during 13 days in culture.

VM-2 antibodies did not affect the growth of keratinocytes over a six-day period whereas VM-1 antibodies did. VM-2 did not inhibit growth of fibroblasts over a five-day period.

B. The specificity of VM-2 antibody was further assessed by cellular ELISA on various cell types as described above. Results are summarized in Table I. VM-2 binds to a determinant on an antigenic site present on SCC, A-431, A-549 and Hela cells. Normal fibroblasts, endothelial cells or cells from the hematopoietic lineage are not recognized by the VM-2 antibody.

C. Upon immunoprecipitation of the VM-2 antibody incubated with lysates of biosynthetically radioactively labeled target cells, 2 protein bands, each of apparent molecular weight of approximately 120,000, were revealed by fluorography. Protein bands of similar molecular weight were obtained from SCC, A-431 and Hela cells, although in different relative amounts. No protein was precipitated from control HFF cells under the same conditions.

0157613

-28-

## Table I

### Binding of VM-2 Antibody to Cells as Determined by Cellular ELISA

| Cell Type | Reactivity with VM-2 Antibody |
|---|---|
| Skin squamous cell carcinoma (human) (SCL-1) | +++ |
| A-431, vulvar carcinoma (human) | ++ |
| A-549, bronchial carcinoma (human) | + |
| Hela, cervical carcinoma (human) | +++ |
| Foreskin fibroblasts (human) | - |
| 3T3 fibroblasts (murine) | - |
| Kidney fibroblasts (rat) | - |
| Peripheral blood lymphocytes (human) | - |
| Peripheral blood monocytes (human) | - |
| Erythrocytes (human) | - |
| Molt, T lymphoma (human) | - |
| Daudi, B lymphoma (human) | - |
| P388 $D_1$, macrophages (murine) | - |
| Aortic endothelial cells (bovine, rabbit) | - |
| Venous endothelial cells (bovine, murine) | - |

At 10 µg/ml VM-2 antibody
+:O.D. > 10 times background
++:O.D. > 20 times background
+++:O.D. > 40 times background

## EXAMPLE 3

### Detection of Residual Tumor Cells in
### Microscopically Controlled Surgery (MCS)

A. Frozen sections of skin were prepared from tissue obtained at surgery, which was snap-frozen on a chuck placed on dry ice using OCT compound (Lab-Tek Division, Miles Laboratories Inc., Naperville, IL). Sections were cut with a microtome. The binding of antibodies to epidermal cells in situ was determined by immunofluorescence staining using rabbit antimouse fluorescein isothiocyanate-conjugated Ig (R/M-FITC). This reagent was adsorbed on dispersed human skin cells.

7995K                                                                24300-FF

Frozen sections of skin were incubated with monoclonal antibody (either VM-1 or VM-2 antibody) in humidified glass petri dishes for 15 min at room temperature, washed with phosphate-buffered saline (PBS), labeled with R/M-FITC for 15 min, and washed with PBS. Sections were covered with fluorescent antibody mounting fluid (Difco laboratories Inc., Detroit, MI) and examined with a Zeiss fluorescence microscope.

B.    Frozen sections of human skin were labeled, using the IP staining technique.  Briefly, the tissue was fixed with acetone, incubated with VM-1 or VM-2 antibody, washed with PBS, incubated with biotin-conjugated goat antimouse IgG (G/M-IgG) (Tago, Inc., Burlingame, CA), washed with PBS, labeled with avidin-conjugated horseradish peroxidase, washed with PBS and $H_2O$, incubated with diaminobenzidine (DAB) solution, and rinsed with PBS and $H_2O$.  All incubations were at room temperature.  The sections were processed in 0.5% $CuSO_4$ solution, counterstained with Giemsa, cleared, and mounted.  ·

C.    Results and Discussion

In tissue from seven of seven different patients with basal cell carcinoma (BCC), including a morphea form tumor, and two patients with squamous cell carcinoma (SCC), VM-1 and VM-2 antibody, respectively, allowed distinct staining of the tumor cells.  Staining was uniform in all of the BCC and in one of the SCC specimens.  In the other SCC, antibody binding was most dense at the periphery of large tumor islands, and less dense in the centers, where the cells appeared more squamoid.  In smaller tumor nests the staining was quite uniform.  Small islands of tumor were easily detectable in all specimens by both IP and IF technics. Control staining with a murine myeloma antibody of the same isotype as VM-1 and VM-2 did not delineate the tumor cells.

## Example 4
### Determination of the Presence of Malignant Cells in Exfoliative Cell Specimens Obtained from the Cervix

Cervical smears were obtained from 20 healthy volunteers on routine gynecological examination and from 20 patients with invasive squamous cervix carcinoma established independently by conventional cytology of biopsies. The smears were acetone dipped for 5 min at room temperature, air "dried" and kept desiccated at -70°C until use. The smears were then covered with 200 μl of 10-50 μg/ml fluoresceinated VM-2 antibody, prepared as described in Example 5 following, for 30 min at room temperature in a humidified chamber. The smears were extensively washed in PBS by transfer in several Coplin jars, mounted in 50% glycerol in PBS and observed with a Zeiss Universal fluorescence microscope. No cellular staining was observed for any of the smears obtained from the healthy volunteers. In smears of 20 out of 20 patients with invasive squamous cervix carcinoma an intense membrane fluorescence staining was observed on small round cells in the sample. Large squamous cervical cells with pycnotic nuclei were not stained, nor were erythrocytes and polymorphs found in most of the samples. For some patients, available duplicate smears were stained according to the conventional Papanicolaou technique. The small round cells stained by the VM-2 antibody were confirmed in these samples as possessing neoplastic features (altered nucleus/cytoplasmic ratio
and basophilic, abnormal nuclei).


Example 5

Conjugation of VM-2 Antibody to Fluorescein

VM-2 antibody (5 mg/ml) was reacted with a tenfold
molar excess of fluorescein isothiocyanate immobilized on
Celite (Molecular Probes) for 30 minutes in the dark at
room temperature in a 0.2 M bicarbonate buffer, pH 9.2.
Excess fluorescein isothiocyanate was removed by gel
chromatography on a Sephadex G50 column in phosphate
buffered saline.  The fluorophore/protein ratio was
calculated from the absorbance of the conjugate at 493 nm
and 280 nm and varied between 2.4 to 4.2 from one
preparation to another.

The antibody conjugates were either kept at 4°C in
the presence of 0.1% merthiolate or frozen in aliquots at
-70°C in the presence of 10% glycerol.


The cell line, designated VM-2, was deposited on
March 21, 1984 at the A.T.C.C. and received accession
number HB8530.  It consists of a hybrid cell combining
normal splenocyte from BALB/c mice, fused with P3 myeloma.


The invention has been described in detail with
particular reference to the above embodiments.  It will
be understood, however, that variations and modifications
can be effected within the spirit and scope of the
invention.

0157613

-32-

CLAIMS:

1. A monoclonal antibody specific for an antigenic site on a protein characteristic of a human basal cell and a malignant squamous cell and useful binding fragments of said antibody.

2. The antibody of Claim 1 which is of the IgG type.

3. The antibody of Claim 1 which does not bind to mesenchymal cells.

4. The antibody of Claim 1 wherein said protein is characterized as having a molecular weight of about 120,000 daltons as determined by polyacrylamide gel electrophoresis.

5. A cell line having the identifying characteristic of expressing the antibody of Claim 1.

6. The monoclonal antibody of Claim 1, which is VM-2 antibody or antibody having functional equivalency with VM-2 antibody.

7. The monoclonal antibody of Claim 1 conjugated to a label capable of producing a detectible signal.

8. A VM-2 murine hybridoma.

7995K                                                      24300-FF

9.    A monoclonal antibody produced by the hybridoma of Claim 8 and the useful binding fragments of said antibody.

10.    A cell line having the identifying characteristic of expressing VM-2 antibody or the functional equivalent thereof.

11.    A method for determining the presence of a malignant condition in tissue, which comprises
        (a)    contacting a specimen of said tissue with an antibody specific for an antigenic site characteristic of a human basal cell, which antibody is capable of binding to basal cells and malignant squamous cells and capable of distinguishing such cells from other cell types in said specimen, under conditions for binding of said antibody to said specimen, and
        (b)    observing the presence of binding of said antibody to said specimen, said binding being related to the presence of a malignant condition in said tissue.

12.    The method of Claim 11 wherein the antibody is VM-2 antibody or a functional equivalent thereof.

13.    The method of Claim 11 wherein the antibody is VM-1 antibody or a functional equivalent thereof.

14. The method of Claim 11 wherein the extent of binding is determined by contacting said section with a conjugate of a label and specific binding partner for said monoclonal antibody, said label being capable of producing a detectible signal.

15. The method of Claim 11 wherein the monoclonal antibody is conjugated to a label capable of producing a detectible signal.

16. The method of Claim 14 wherein the labeled specific binding partner for said monoclonal antibody is an antibody specific for said monoclonal antibody.

17. A method according to anyone of Claims 11 to 16, for detecting residual tumor cells in skin subjected to microscopically controlled surgery to remove a tumor, which method comprises -

(a) contacting a section of skin adjacent said tumor with the monoclonal antibody of Claim 1 under conditions for binding of said antibody to said residual tumor cells and

(b) determining the presence of immune complexes of said monoclonal antibody, the presence of immune complexes of said antibody being related to the presence of residual tumor cells in said skin.

18. A diagnostic kit, comprising -

(a) the monoclonal antibody of Claim 1, and

(b) a conjugate of (1) a label which is a member of a signal producing system and (2) a specific binding partner of the monoclonal antibody of Claim 1.

19.  A diagnostic kit, comprising -

a conjugate of (1) a label which is a member of a signal producing system and (2) the monoclonal antibody of Claim 1.

20.  A method for determining the presence of a malignant condition in an exfoliative cell specimen obtained from a locus of interest, which method comprises -

(a)  contacting said exfoliative cell specimen with an antibody specific for an antigenic site that is usually only found in said specimen when a malignant condition is present, which antibody is capable of distinguishing said antigenic site from other antigenic sites which are usually found in said specimen, under conditions for binding of said antibody to said antigenic site to form immune complexes, and

(b)  observing the presence of said immune complexes, the presence of said immune complexes being related to the presence of a malignant condition at said locus.

21.  The method of Claim 20 wherein said antigenic site shares determinants with an antigenic site present on a normal cell that is not usually found in said specimen.

22.  The method of claim 20 wherein said antibody is specific for a protein on an antigenic site characteristic of normal epidermal basal cells.

23.  The method of claim 20 wherein the protein has a molecular weight of about 120,000 by one dimensional gel electrophoresis.

24.  A method according to Claim 20 for detecting the presence of neoplastic cells in an exfoliative cell specimen obtained from a locus of interest, which comprises -

(a)  contacting said exfoliative cell specimen with an antibody specific for an antigenic site on a protein characteristic of a human basal cell and a malignant squamous cell under conditions for binding of said antibody to said antigenic site and

(b)  observing the presence of binding of said antibody to said antigenic site, the binding of said antibody being related to the presence of neoplastic cells in said specimen.

25.  The method of anyone of Claims 20 to 24 wherein said antibody is a monoclonal antibody.

26.  The method of claim 25 wherein said antibody is VM-2 antibody or the functional equivalent of VM-2 antibody.

27.  The method of anyone of claims 20 to 26 wherein said locus is a locus having membranes covered with squamous cells.

28.  The method of anyone of claims 20 to 27 wherein said antibody is conjugated to a label capable of producing a detectable signal.

7995K                                                      24300-FF

-37-

29.  The method of anyone of claims 20 to 28 wherein said locus is the cervix.

30.  The method of anyone of Claims 20 to 28 wherein said locus is the lung.

31.  A method according to Claim 20 for detecting a malignant condition in the cervix, which comprises -

(a)  contacting an exfoliative cell specimen obtained from said cervix with an antibody which binds to a protein on an antigenic site present in said cell specimen only when a malignant condition exists, which protein is characterized as having a molecular weight of about 120,000 as determined by one dimensional gel electrophoresis under conditions for binding of said antibody to said antigenic site and

(b)  observing the presence of binding of said antibody to said antigenic site, the binding of said antibody being related to the presence of a malignant condition in the cervix.

32.  The method of Claim 31 wherein said antibody is VM-2 antibody or the functional equivalent of VM-2 antibody.

33.  The method of claims 31 or 32 wherein the binding is determined by contacting said specimen with a conjugate of a label and a specific binding partner for said antibody, said label being capable of producing a detectable signal.

7995K                                            24300-FF

34.    A diagnostic kit, comprising -

(a)    a conjugate of (1) a label which is a member of a signal producing system and (2) a monoclonal antibody specific for an antigenic site on a protein characteristic of a normal cell, which normal cell shares determinants with an antigenic site that is found in an exfoliative cell specimen only when a malignant condition is present and

(b) means for obtaining said exfoliative cell specimen.

35.    A diagnostic kit, comprising -

(a)    a monoclonal antibody specific for an antigenic site on a protein characteristic of a normal cell, which normal cell shares determinants with an antigenic site that is found in an exfoliative cell specimen only when a malignant condition is present,

(b)    a conjugate of (1) a specific binding partner of said monoclonal antibody and (2) a label which is a member of a signal producing system, and

(c)    means for obtaining said exfoliative cell specimen.

36.    A diagnostic kit according to anyone of the Claims 18, 19, 34 and 35, wherein said antibody is VM-2 antibody or the functional equivalent of VM-2 antibody.